**Europäisches Patentamt**

(19) **European Patent Office**

**Office européen des brevets**

(11) Publication number : **0 343 239 B1**

(12) # EUROPEAN PATENT SPECIFICATION

(45) Date of publication of patent specification :
20.01.93 Bulletin 93/03

(51) Int. Cl.⁵ : **A61M 31/00, B27N 3/00**

(21) Application number : **89901193.6**

(22) Date of filing : **08.11.88**

(86) International application number :
**PCT/US88/03986**

(87) International publication number :
**WO 89/04689 01.06.89 Gazette 89/12**

(54) GLASSY MATRIX FOR ADMINISTRATION OF BENEFICIAL AGENT.

(30) Priority : **16.11.87 US 120892**

(43) Date of publication of application :
**29.11.89 Bulletin 89/48**

(45) Publication of the grant of the patent :
**20.01.93 Bulletin 93/03**

(84) Designated Contracting States :
**BE CH DE FR GB IT LI NL SE**

(56) References cited :
EP-A- 0 059 694
WO-A-86/00004
CH-A- 497 181
FR-A- 2 510 413
US-A- 25 129
US-A- 1 541 744
US-A- 2 996 431
US-A- 3 365 365
US-A- 3 558 768
US-A- 4 130 647
US-A- 4 235 236
US-A- 4 257 426
US-A- 4 381 776
US-A- 4 511 352
US-A- 4 552 555
US-A- 4 684 516

(56) References cited :
US-A- 4 753 800
US-A- 4 758 430
Transfusion, Volume 24, no. 6, issued 1984
(Philadelphia), B.A. Myhre et al.:
"Preservation of red cell antigens duringstorage of blood with different anticoagulents"

(73) Proprietor : **BAXTER INTERNATIONAL INC. (a
Delaware corporation)**
**One Baxter Parkway**
**Deerfield Illinois 60015 (US)**

(72) Inventor : **BREILLATT, Julian, P.**
**933 Highland Road**
**Mundelein, IL 60060 (US)**
Inventor : **WOO, Lecon**
**1013 Shari Lane**
**Libertyville, IL 60048 (US)**
Inventor : **NELSON, Deanna, J.**
**729 Thomas Court**
**Libertyville, IL 60048 (US)**
Inventor : **APPL, Richard**
**1111 Robey Avenue**
**Downers Grove, IL 60068 (US)**

(74) Representative : **MacGregor, Gordon et al**
**ERIC POTTER & CLARKSON St. Mary's Court**
**St. Mary's Gate**
**Nottingham, NG1 1LE (GB)**

## Description

TECHNICAL FIELD

As blood is collected from the donor, it passes into a container such as a blood bag which contains an anticoagulant system. Such anticoagulant systems are typically a small amount of liquid solution which is stored in the bag, being tppically ACD, CPD, CPD-adenine, or the like. Additionally, it has been suggested that the anticoagulant spstem may be placed as a dried coating on the interior of the blood collection container.

With these systems, as the blood is introduced into the container, the ratio of the whole blood present to the anticoagulant present is undesirably high, consequently producing a significant and detrimental osmotic imbalance. While this problem is quickly corrected with the addition of subsequent amounts of blood, it can be damaging to the first aliquot of blood cells administered into the collection bag.

Accordingly, there is a need for a blood handling spstem where beneficial agents such as anticoagulant/storage agents may be administered to the blood without the initial, undesirable osmotic effect that currently takes place at the beginning of the blood collection.

Additionally, in the manufacture of conventional blood storage bags which contain a liquid anticoagulant, a capital intensive and closely controlled manufacturing process is required, including a liquid filling operation (which requires a clean room), batch steam sterilization procedures, and packaging of the blood bag in an additional moisture barrier container for storage. If it were possible to make use of blood bags which did not contain a liquid anticoagulant system, it would be possible to greatly simplify the manufacturing process, making use of continuous radiation sterilization, and eliminating the additional moisture barrier packing which is currently necessary.

Additionally, blood storage bags which contain either a liquid anticoagulant or a dried coating thereof on the interior walls result in a container that must be completely filled with blood in order to be usable. If, for any reason, the donor is unable to donate one complete unit of blood, the entire, underfilled unit typically is not used, due to an excess concentration of anticoagulation agent present in the reduced volume of blood, since the anticoagulant present in the bag is intended for one complete unit of blood. If empty, anticoagulant-free blood storage bags were used in which the blood was still properly provided with anticoagulant/storage agents, these underfilled units could still be used for pediatric or partial transfusion purposes, and would not have to be discarded.

In a United States patent application entitled Controlled Administration of Beneficial Agent to Blood, assigned to Baxter Travenol Laboratories, Inc. and filed on the same day as this present application, a technique and apparatus for solving the above problems is provided, to permit the application of a beneficial agent such as anticoagulant/storage agent to blood while the blood is passing through a conduit into a container. Thus, blood storage containers without any beneficial agent therein may be manufactured and used, for the significant manufacturing advantages described above. Additionally, partial units of blood may be collected and used.

As described in the patent application cited above, the beneficial agent may be carried in a conduit through which medical solution, tppically blood, passes, typically on its way to a container, As it passes through the beneficial agent, a controlled amount of beneficial agent dissolves into each aliquot of medical solution which passes. In accordance with this invention, improved uniformity of transfer of the beneficial agent to the various aliquots of medical solution may be obtained, along with desirable improvement in control and precision in the process.

Additionally, the controlled or sustained delivery of chemical agents or drugs is commonly obtained by diffusion from a permanent matrix that contains and serves as a reservoir of the agent. In some cases, the porosity of the matrix fixes the diffusion rate of the chemical into the surrounding environment. In other cases, a rate-limiting membrane is juxtaposed between the reservoir and the target. Examples include pesticide dissemination, transdermal drug delivery and administration of therapeutic agents into parenteral fluids or blood.

In the case of delivery into blood or parenteral fluids, the introduction of a permanent matrix entails an additional material whose biocompatibility must be assured, and that will not shed particles into the circulation. Moreover, it is difficult to determine when a permanent matrix has delivered its total dose of entrapped agent to a parenteral fluid or to blood, since neither the visual appearance nor the density of the matrix will necessarily change with depletion of the agent. Further, because matrices allowing outward diffusion of the agent must have a finite porosity, they cannot act as a barrier to moisture or gas diffusion into the matrix and the agent formulation therein entrapped.

The ideal matrix for parenteral or blood delivery would be one that protects and stabilizes the active agent in the dry state, then disappears as the agent is delivered into the flowing stream. The matrix could be the agent itself or a component thereof, a beneficial metabolite, or a non-toxic, excretable molecular species that dissolves in the stream of parenteral fluid or blood passing over it, thus releasing the entrapped agent.

Ideally, the matrix dissolution rate determines the delivery rate of the active agent; as the matrix dissolves and disappears, the agent is contacted by the surrounding fluid and itself dissolves. However, one of the factors limiting the delivery rate of the agent into the fluid stream is its own solubility. In the extreme case the matrix could dissolve away leaving the agent as an insoluble mass in the fluid. To ensure that the dissolution of the agent is equal to or greater than that of the matrix, chemical buffers or reactants can be entrapped in the matrix with the agent, under conditions that they would not react during the formation of the filled matrix, but such that when the dissolving fluid reaches the agent, a localized environment will be established that favors its dissolution.

Alternately, an agent may be stable to storage only in a pharmacologically inactive form. Chemical reactants that transform the pro-drug into its active form may be co-entrapped in the matrix. As the fluid dissolves the matrix and its contents, a high concentration of the various dissolving chemical species will occur at the solid-liquid interface, providing conditions that enhance the desired chemical transformation.

In this invention, a matrix is provided for delivery of anticoagulant during blood collection, for parenteral drug delivery, and for any other controlled release of beneficial agent into a fluid stream.

DESCRIPTION OF THE INVENTION

In this invention, a method is provided for passing solution, tppically medical solution such as blood, any desired parenteral solution, enteral nutrient solution, or the like, through a conduit, typically into a container or the circulatory system, the digestive spstem, the peritoneal cavity, or the like. In accordance with this invention, one passes the medical solution in the conduit across a solid mass of beneficial agent, wherein the beneficial agent is typically dispersed within a substantially anhydrous glassy sugar matrix, although the beneficial agent may be the glassy sugar matrix itself.

The term "sugar" shall include monosaccharides or polymers thereof having a degree of polymerization (d.p.) of preferably about 1 or 2, but optionally up to the hundreds. The term "sugar" also includes the correspnonding sugar alcohols. The term "glass" or "glassy" refers to the amorphous, non-crystalline form assumed by viscous sugars, although local areas of crystalization may occur within such a glassy matrix without impairing its overall classification as a glass.

US-A-1541744 discloses a method for producing a tablet that will dissolve quickly and completely in the barrel of a hypodermic needle for subsequent administration.

EP-A-0059654 discloses a method for controlled administration of a beneficial agent to a medical fluid in which said fluid is passed over a solid mass which contains the beneficial agent.

The pre-characterising part of claim 1 is based on EP-A-0059654, and the distinguishing features of the present invention are set out in the characterising part of claim 1.

The mass of beneficial agent may, in one embodiment, be shaped and proportioned whereby a controlled amount of the beneficial agent dissolves into passing medical solution in a manner that is substantially uniform or otherwise predetermined. As a result of this, no substantial portion of the medical solution needs to be exposed to a significantly higher concentration of the beneficial agent than other portions of the medical solution.

For example, the tablet of beneficial agent used herein may comprise a sugar selected from the group consisting of glucose, lactose, sorbitol, fructose, and xylitol in a substantially anhydrous glass form, the tablet also including at least one added effectlive agent for dissolving in medical solution as the sugar dissolves in the medical solution.

In one typical use of the invention of this application, the medical solution may be blood, for example blood which is in the process of being collected from a donor and passed into the donor bag of a single or multiple blood bag spstem which may be of conventional design and use, but for the modifications provided by this invention, in this circumstance, the beneficial agent may include an anticoagulant uhich is dispersed in the sugar glass. Any known anticoagulant or other blood cell preservative, such as platelet preservatives, may be so incorporated into such a sugar glass. For example, the sugar that forms the glass may be glucose, which may also include a water soluble, non-toxic source of citrate in sufficient concentration to have an anticoagulating effect on passing blood. Additionally, a water soluble, non-toxic source of phosphate may be present in a concentration effective to promote blood storage in known manner. Adenine may be present, if desired, as well as any other ingredient which is desired for the storage of blood or components thereof.

By the term "blood", it is intended to include not only whole blood, but components of blood as may be desired, for example blood plasma (either platelet rich or platelet poor) or suspensions of packed red cells, white cells, or platelets in saline solution, any other appropriate suspension of blood cells, or the like.

Typical sugars which may be used are, of course, soluble, non-toxic sugars which may or may not be metabolizable as a nutrient according to the desires of the formulator and the use to which the glassy mass of beneficial agent is to be put. Specifically, the sugar may be one or more of those selected from the group described above.

Examples of usable citrates and phosphates which may be used in the manner described above include sodium citrate, citric acid, and monosodium or disodium phosphate, The proportions of sodium citrate and citric acid may be adjusted to obtain the pH desired.

The glassy mass of beneficial agent is preferably provided in the form of one or more dry tablets, with means provided in the conduit for carrying the solid mass of beneficial agents and permitting blood or other medical solution to pass through the conduit to enter into contact with the solid mass. As solution passes through the solid mass, it picks up a substantially predetermined amount of beneficial agent, and carries it away. The specific geometry of the preferred tablet may be specifically shaped to provide a desired, controlled, relatively constant release of the beneficial agent as the solution passes across it. Specifically, a single tablet may be provided which is relatively long and wide relative to its thickness. With such a configuration, as the tablet erodes, there is little change in the surface area of the tablet, so that the amount of beneficial agent which dissolves into passing medical solution remains substantially uniform, at least until the tablet begins to disintegrate. One may have the tablet or tablets of a desired size and thickness so that the last of the medical solution has passed through the conduit before the tablet or tablets erode away so much that they begin to break up, at which point a significant difference in the transfer rate of beneficial agent into the solution can take place. Otherwise, the tablet may be proportioned to completely dissolve before flow terminates to provide visual indication of complete dosage.

The dry, glassy beneficial agent may be a molded or pressed tablet structure, which may be flat and thin as described above, or the tablet may be a pressed, solid structure having multiple flow channels through it for flow of medical solution therethrough, or a cylinder positioned so that medical solution flows through its bore, or any other shape or structure as desired.

Since the geometry, state, and size of the glassy mass of beneficial agent can be controlled, improvements in control of transfer of the beneficial agent to the medical solution are obtained. For example, a variable rate of dosage may be provided by the shape of the tablet, if desired. A large, initial bolus dose may be provided, for example, if the outer portion of the tablet is more porous than an inner portion thereof.

When the beneficial agent desired is an anticoagulant/preservative preparation, it may comprise a solid mass or tablet of a sugar such as dextrose, formed in the shape as described above, with the solid, glassy mass containing an effective anticoagulating amount of a soluble, non-toxic citrate, as described above, so that the dextrose and the citrate dissolve together in a controlled release manner in the passing blood. Similarly, the anticoagulant/preservative formulation may include an amount of soluble, non-toxic phosphate as stated above.

Thus, the use of the substantially dry glassy mass of beneficial agent, positioned in a conduit, provides significant advantages. The specific advantages of the empty bag or the receptacle which thus may be used for blood or other medical solution have been described above. Additionally, transportation and handling advantages are achieved, when compared particularly with containers of frozen drug solution, which must be transported in frozen condition. In many cases, the drug is simply incorporated into the glassy mass of sugar. If desired, a parenteral solution such as saline may be passed over the glassy mass and directly into the patient, carrying the dissolved sugar and a controlled amount of drug with it. Thus, critical drugs may be administered to a patient without a special step of reformulation of the dry drug into a parenteral solution.

Thus, significant advantages of manufacturing, transportation, storage, and handling are provided in accordance with this invention.

Additionally, sugar glasses having a low water content (for example 1 to 4 percent by weight) are good oxygen barriers. Thus, oxygen-sensitive drugs may be protected while incorporated in the glassy mass of beneficial agent used in this invention. Additionally, the spstem of this invention is well suited for gamma irradiation sterilization, and moisture sensitive drugs are also protected by storage in the glassy mass of beneficial agent in accordance with this invention. It is believed that the sugar present can exert a protective action on drugs or other effective agents which are mixed in the glassy mass with the sugar prior to contact with the medical solution for use, providing protection against moisture, oxygen, and even gamma radiation, to reduce loss of effective agent during gamma irradiation sterilization.

Additionally, the glassy sugar masses used in this invention are resistant to bacterial attack due to their low water content. It is generally preferred for the water content of such glassy masses to be about 1 to 2 percent, and tppically no more than about 5 percent by weight.

Also, the glassy mass of this invention can be effectively molded during manufacture to form a tablet or tablets of desired dimensions to obtain the exact, desired dissolution rate as medical or other solution passes in contact therewith.

Enough amorphous, glassy sugar or sugar alcohol should be present to serve as a binder for other materials present. Typically, at least about 20 weight percent of amorphous, glassy sugar is present in the dry, beneficial agent of this invention, surrounding and protecting other inclusions therein.

If desired, a substantially pure glassy sugar mass may be used as the beneficial agent. Tablets of such material may be used to form glucose parenteral solutions at their site of use, for example, from sterile water at the site, to avoid transporting the water from elsewhere.

Other ingredients such as soluble fillers and coloring agents may also be present in the glassy mass.

The glassy mass of this invention may be manufactured by dispersing a substantially dry, powdered beneficial agent into a substantially dry, soluble fluid phase material and thereafter allowing the dispersion thus formed to assume a solid state. The fluid phase material may be a molten sugar, especially those listed above, sodium citrate, citric acid, mannitol, or mixtures thereof, for example. The molten material may be heated to dryness. Then, the dry, powdered beneficial agent is added, being, for example, powdered sugar to promote solidification, A drug or the like may also be used as the powdered beneficlal agent, Then the dispersion is-allowed to cool and solidify, typically being molded into tablet form for use as described herein.

DESCRIPTION OF DRAWINGS

Fig. 1 is a plan view of a blood collecting set made in accordance with this invention, shown to be integrally connected to a blood storage bag; and
Fig. 2 is a sectional view taken along line 2-2 of Fig. 1.

DESCRIPTION OF SPECIFIC EMBODIMENTS

Referring to the drawings, there is shown a set 10 for collecting blood from a patient and conveying it to blood storage container 12, which may be made in substantially conventional manner. Set 10 carries at one end a conventional blood connection needle 14 for collecting blood from the venous system of the patient, which blood then passes through set 10 into blood bag 12.

In accordance with this invention, the conduit of set 10 defines an enlarged portion 16 which includes a glassy, solid mass 18 of dry blood storage anticoagulant preparation, typically having a water content on the order of no more than about 2 percent by weight. Anticoagulant tablet 18 is shown to be made in the form of a thin, rectangular member positioned within enlarged portion 16. The inner walls of both sides of enlarged portion 16 define an array of small projections 20 which project inwardly to define flow passages between the inner wall of enlarged portion 16 and the surface of anticoagulant preparation tablet 18.

Specifically, anticoagulant preparation tablet 18 may comprise, for example, a dried mixture of:

| | Wt. % Present |
|---|---|
| Dextrose | 44 |
| Sodium Citrate | 46 |
| Citric Acid | 6 |
| Sodium Biphosphate | 4 |

The above dextrose is dissolved as a near saturated solution in water by heating to boiling. Then, water is removed by boiling under a vacuum of about 2 inches of mercury and up to about 310 degrees F., until the dextrose reaches the desired water content of 2 weight percent or less (as defined by its temperature at the air pressure obtained by the vacuum system). The viscous mass is then cooled to avoid crystallization, to obtain a supercooled solution.

The other ingredients are added when the temperature of the molten dextrose falls to about 200 degrees F., with vigorous stirring or alternatively in a twin screw extruder, to form a homogeneous mass. This material is molded into the desired shape and allowed to solidify, to form a white, glassy mass in the shape of tablet 18, which may be then placed in portion 16.

Blood bag 12 may be initially free of any anticoagulant. The entire system, including tablet 18, is tppically radiation sterilizable, since it is substantially free of moisture. Additionally, no overpouch or other outer package is required to prevent the loss of water from a liquid anticoagulant present, which constitutes a significant advantage over the conventional present blood collection equipment.

For use, a conventional phlebotomy is made into a vein of a blood donor with needle 14, and the blood flows through conduit 10. As it flows, it enters enlarged portion 16, where it flows in a pair of opposed flow paths 22 between the walls 24 of enlarged portion 16 and the flat surfaces of tablet 18. As the blood so flows, the substance of tablet 18 is dissolved away into the flow blood at a relatively uniform rate of dissolution, so that each individual portion of the flowing blood passing through conduit 10 picks up a substantially similar amount of the dissolved substance of tablet 18 as it flows across it. From there, the flowing blood, carrying the dissolved anticoagulant, glucose, etc. passes into bag 12 for storage, without encountering an excessive concentration of anticoagulant or other agent.

Other dry forms of beneficial agents, particularly anticoagulant/preservatives, may also be used as desired, as a substitute for the specific forms of beneficial agent illustrated above with respect to tablet 18.

Any design of blood bag or other container may also be utilized in accordance with this invention. If desired, the tablet 18 or an equivalent mixture of materials may be encapsulated and released via diffusion through a semipermeable membrane.

Additionally, the invention of this application may be used in conjunction with various apheresis procedures, for example bag apheresis hardware collection, apheresis, or continuous apheresis procedures. For example, medications may be administered to blood in accordance with this invention by an apheresis procedure or the like.

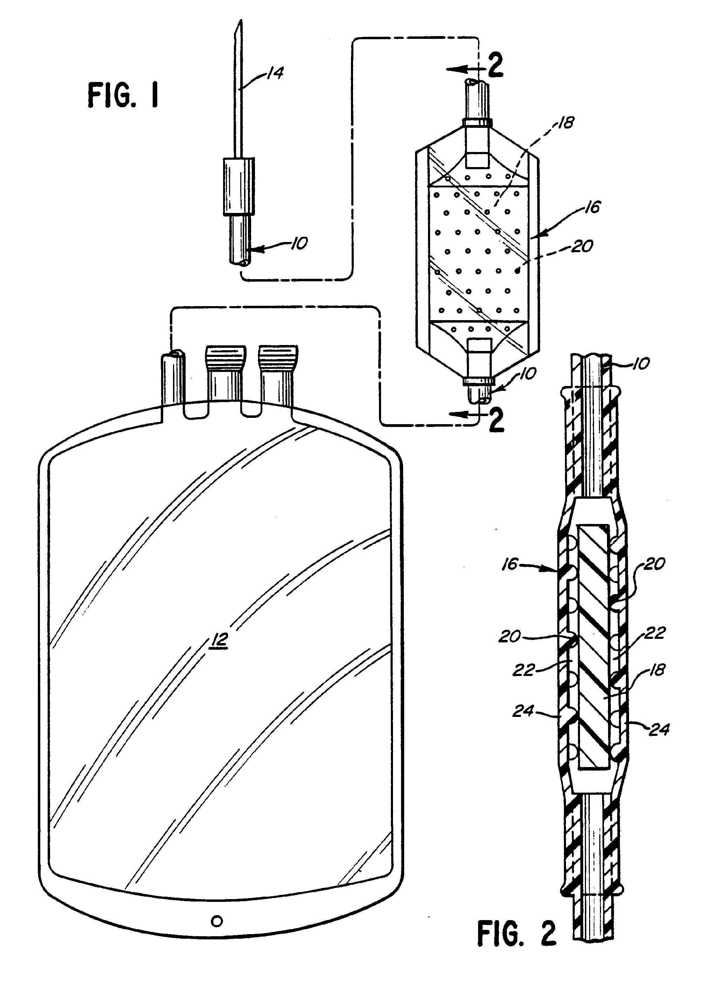

Alternatively, an antibiotic or other drug may be mixed into the molten sugar when it is at a temperature of about 200 degrees F. or below. For example, aspirin may be added to dextrose sugar glass. Antibiotics and other peptides may be added with advantage to sorbitol glass. After intimate mixing, the molten mixture is molded into tablet form, or any other desired shape, and allowed to solidify to a solid, glassy form. Such glassy tablets may be stronger than compressed powder tablets, having more uniform dissolution rates for more accurate dosage, and higher dissolution rates than compressed powder tablets with conventional binders. The resulting tablets may be placed into a chamber similar to enlarged portion 16 of set 10, which may be adapted for connection, or integrally connected with, a bag of parenteral solution. Fig. 1 in this circumstance can represent such a system, in which bag 12 contains, for example, sterile normal saline solution, or even pure water, although such a latter system might not be fail safe since intravenously administered water is very damaging to blood cells.

In this instance, connection with the venous system of a patient is made by needle 14, and the parenteral solution in bag 12 is allowed to pass from bag 12 through set 10 into the patient's venous system. As it passes through enlarged portion 16, the sugar and admixed drug of table 18 dissolves into the passing parenteral solution at a predetermined rate, so that the desired amount of drug may be administered simply by administering a desired quantity of parenteral solution from bag 12 at a predetermined rate of flow.

Thus, the invention of this application may not only be used for the handling of blood, but it may be used for the administration of drugs or other beneficial agents directly to the patient, or to another container in which solution from a bag is passed through a chamber which contains a glassy mass of a sugar, containing the desired beneficial agent. Such a system is very flexible as to the amount of beneficial agent which is delivered, contrary to many present systems. If one wishes to deliver only one third of the available beneficial agent in tablet 18, one can simply administer a predetermined amount of solution from bag 12 through set 10 to the patient, and then terminating such administration when the desired amount of beneficial agent has been delivered. Additionally, the enlarged chamber 16 may be manufactured in a form of a cartridge for connection to parenteral solution sets, with the various cartridges carrying different types of drugs as may be desired in the glassy mass of sugar. The doctor then needs to only select a cartridge which carries the drug of the desired type and concentration, and administer it to the patient without a special drug reconstitution step previous to the administration.

Other examples of drugs which may be inserted into the glassy mass of this invention include anti-clotting agents such as heparin, antibiotics of any type such as gentamycin, penicillin, or erythromycin, other strongly biologically active drugs that require controlled administration to the patient such as thyroid hormones or insulin, and the like.

Not only can the glassy mass of this invention be stored in a tubular set, but the glassy mass may be placed within an empty bag or other storage container, to dissolve into an aliquot of solution that passes into the container, should that alternative technique be deemed desirable.

Added specific examples are found below showing the manufacture of specific glassy, sugar tablets containing beneficial agents.

As an example of how acetylsalicylic acid may be incorporated into a glassy dextrose tablet, 250 grams of dextrose monohydrate may be dissolved in water at a near saturated solution and boiled to about 250 degrees F. under vacuum of about two inches of mercury, until the dextrose reaches the desired water content of 2-4 wt. percent or less in the manner previously described, but remains in liquid form.

A well-mixed powder containing 78 grams of anhydrous sodium citrate and 55 grams of acetylsalicylic acid is then dispersed in the dextrose liquid after it has cooled to about 200 degrees F., with vigorous stirring or alternatively in a twin screw extruder, to form a homogenous mass. This material is melded into the desired shape and desired to solidify to form tablets 18 of a shape desired. These tablets may then be placed in portion 16 of an administration set, or otherwise used.

When water flows over a tablet containing aspirin dispersed alone in a dextrose matrix, the dextrose matrix dissolves, leaving an undissolved mass of aspirin, since aspirin has a low solubility in water. The sodium citrate ingredient is thus included to continuously establish an acidic pH (4,9-5.0) at the surface of the dissolving tablet, under which conditions the aspirin is readily soluble, and dissolves at a comparable rate to the matrix. This mixture of aspirin and citrate is only possible in an anhydrous system since aspirin reacts with citrate if they are heated together in the presence of a significant amount of water. Also, the aspirin will deacetplate in the presence of water. Thus, by this method, aspirin and citric acid are incorporated together into a glassy tablet, for later i.v. or other administration while strongly suppressing undesired reactions.

Preferably, 25 parts by weight of sugar such as dextrose may be reduced to fluid, anhydrous form according to this invention, and the temperature reduced to 180-240 degrees F.. From 6-10 parts by weight of sodium citrate and from 4-7 parts by weight of acetylsalicylic acid may then be added to the liquid, anhydrous dextrose, followed by mixing and formation by molding of tablets for use in accordance with this invention.

As another example of the manufacturing of another kind of tablet 18 which may be used in the manner previously described, 250 grams of sorbitol is dissolved in water by boiling to 392 degrees F., followed by cooling to about l00-150 degrees F. There is then added to the fluid sorbitol 100 mg. of freeze-dried, powdered peptide mixed with 25 grams of powdered sorbitol as a dispersing agent. The mixture is rendered homogeneous by vigorous stirring, or by twin screw extruder or the like. This material may be formed by molding into tablets or the like, as desired, for use in controlled dosage administration, intravenously or otherwise, of the peptide.

The peptide may be substantially any bioactive protein or polypeptide, for example, antibiotics or the like. Additionally, other low dosage, biologically active materials may be similarly formed into tablets in highly diluted form for similar administration.

One advantage of this technique is that sorbitol or mixtures thereof with other sugars may be cooled to below 200 degrees F. in anhydrous form while retaining significant fluid characteristics, to permit the physical mixing of a heat sensitive desired ingredient. Thus, temperature sensitive ingredients may be administered in accordance with this invention by the use of sorbitol or other materials in accordance with this invention.

Accordingly, by the invention of this application, a system is provided in which a solid drug or other beneficial agent can be reconstituted into its beneficial form at the site of use without an intermediate diluent, if desired. In other words, the beneficial agent of this invention can be directly reconstituted into blood, where its beneficial action takes place directly on the blood without the need of an intermediate diluent. On the other hand, the glassy mass tablet of this invention may be reconstituted into a diluent, for purposes of drug delivery or the like, if desired.

The above has been offered for illustrative purposes only, and is not intended to limit the scope of the invention of this application, which is as defined in the claims below.

## Claims

1. A method for controlled administration of a beneficial agent to a medical fluid, in which said fluid is passed over a solid mass which contains the beneficial agent, and characterised in that said mass comprises a sugar in a substantially anhydrous glass form for providing a predetermined rate of dissolution of the beneficial agent in said fluid.

2. A method according to claim 1, wherein said solid mass is long and wide relative to its thickness and/or having portions of differing porosity whereby a controlled amount of the beneficial agent dissolves into passing medical solution in a manner that is substantially uniform or otherwise predetermined.

3. The method of Claims 1 or 2 in which said medical solution is blood, and said beneficial agent includes an anticoagulant mixed in the solid mass.

4. The method of Claim 3 in which said anticoagulant is a water soluble, non-toxic source of citrate in sufficient concentration,

5. The method of Claim 3 in which said beneficial agent also includes a water soluble, nontoxic source of phosphate in a concentration effective to promote blood storage.

6. The method of Claim 1, 2, 3, 4 or 5 in which said sugar is selected from glucose, lactose, sorbitol, fructose, and xylitol.

7. The method of Claim 1,2,3 or 5 in which said added beneficial agent is drug.

8. The method of Claim 7 in which the drug is sensitive to at least one of moisture, oxygen, and gamma radiation, whereby said sugar exerts protective action on said beneficial agent prior to contact with said medical solution.

9. The method of Claim 1 in which said medical solution is an artificial parenteral solution.

10. The method of Claim 1 in which said beneficial agent is substantially pure sugar defining said mass.

11. A tablet for use in the method of Claim 1, which comprises glucose, in a substantially anhydrous, glass form, and also includes a water soluble, non-toxic source of citrate in sufficient concentration to have an anticoagulating effect on passing blood.

12. The tablet of Claim 11 which includes a water soluble, non-toxic source of phosphate in a concentration effective to promote blood storage.

13. The table of Claim 12 which contains approximately 44 weight percent dextrose, 46 weight percent citrate, 6 weight percent of citric acid, and 4 weight percent of sodium biphosphate.

14. The tablet of Claim 11 in which said tablet contains mixed therewith from 6 to 10 parts by weight of sodium citrate and 4 to 7 parts by weight of acetylsalicylic acid per 25 parts by weight of dextrose present.

15. A tablet for use in the method of Claim 1 which comprises sorbitol in a substantially anhydrous glass form and said tablet contains admixed therewith a powdered peptides.


**Patentansprüche**

1. Verfahren zur kontrollierten Verabreichung eines Heilmittels in ein medizinisches Fluid, wobei das Fluid über eine feste Masse, die das Heilmittel enthält, geführt wird, dadurch gekennzeichnet, daß die Masse einen Zucker in einer im wesentlichen wasserfreien Glasform enthält, welche für eine vorgegebene Geschwindigkeit der Auflösung des Heilmittels im Fluid sorgt.

2. Verfahren nach Anspruch 1, bei welchem die feste Masse im Vergleich zu ihrer Dicke lang und breit ist und/oder Bereiche unterschiedlicher Porosität aufweist, wobei eine kontrollierte Menge des Heilmittels sich in einer vorbeiströmenden medizinischen Lösung in im wesentlichen gleichmäßiger oder anderweitig vorgegebener Weise auflöst.

3. Verfahren nach den Ansprüchen 1 oder 2, bei welchem die medizinische Lösung Blut ist und das Heilmittel ein in die feste Masse eingemischtes Antikoagulans enthält.

4. Verfahren nach Anspruch 3, bei welchem das Antikoagulans eine wasserlösliche, nicht-toxische Quelle für Zitrat in ausreichender Konzentration ist.

5. Verfahren nach Anspruch 3, bei welchem das Heilmittel außerdem eine wasserlösliche, nicht-toxische Quelle für Phosphat in einer zur Verbesserung der Blutlagerung wirksamen Konzentration enthält.

6. Verfahren nach den Ansprüchen 1, 2, 3, 4 oder 5, bei welchem der Zucker aus der Gruppe gewählt wird, die Glukose, Laktose, Sorbitol, Fruktose und Xylitol umfaßt.

7. Verfahren nach den Ansprüchen 1, 2, 3 oder 5, bei welchem das zugesetzte Heilmittel ein Medikament ist.

8. Verfahren nach Anspruch 7, bei welchem das Medikament zumindest gegenüber Feuchtigkeit oder Sauerstoff oder Gammastrahlung empfindlich ist, wobei vor Kontakt mit der medizinischen Lösung der Zucker auf das Heilmittel eine Schutzwirkung ausübt.

**9.** Verfahren nach Anspruch 1, bei welchem die medizinische Lösung eine künstlich hergestellte Lösung zur parenteralen Verabreichung ist.

**10.** Verfahren nach Anspruch 1, bei welchem das Heilmittel im wesentlichen reiner Zucker ist, der die Masse bestimmt.

**11.** Tablette zur Verwendung bei dem Verfahren nach Anspruch 1, die Glukose in einer im wesentlichen wasserfreien Glasform enthält und außerdem eine wasserlösliche, nicht-toxische Quelle für Zitrat in ausreichender Konzentration enthält, damit auf vorbeiströmendes Blut eine Antikoagulierwirkung erzielt wird.

**12.** Tablette nach Anspruch 11, die eine wasserlösliche, nicht-toxische Quelle für Phosphat in einer zur Verbesserung der Blutlagerung wirksamen Konzentration enthält.

**13.** Tablette nach Anspruch 12, die etwa 44 Gew.% Dextrose, 46 Gew.% Zitrat, 6 Gew.% Zitronensäure und 4 Gew.% Natriumbiphosphat enthält.

**14.** Tablette nach Anspruch 11, bei welcher die Tablette darin eingemischt 6 bis 10 Gewichtsanteile Natriumzitrat und 4 bis 7 Gewichtsanteile Acetylsalicylsäure auf je 25 Gewichtsanteile vorhandener Dextrose enthält.

**15.** Tablette zur Verwendung bei dem Verfahren nach Anspruch 1, die Sorbitol in im wesentlichen wasserfreier Glasform enthält und welche ein darin eingemischtes pulverförmiges Peptid enthält.

## Revendications

**1.** Procédé destiné à une administration contrôlée d'un agent utile dans un fluide médical, dans lequel ledit fluide est passé sur une masse solide qui contient l'agent utile, caractérisé en ce que ladite masse comprend un sucre sous une forme vitreuse sensiblement anhydre permettant de fournir un débit prédéterminé de dissolution de l'agent utile dans ledit fluide.

**2.** Procédé selon la revendication 1, dans lequel ladite masse solide est longue et large par rapport à son épaisseur et/ou elle comporte des parties à porosités différentes, d'où il résulte qu'une quantité contrôlée de l'agent utile se dissout dans la solution médicale qui passe d'une manière qui est sensiblement uniforme ou sinon prédéterminée.

**3.** Procédé selon la revendication 1 ou 2, dans lequel ladite solution médicale est du sang et ledit agent utile inclut un anticoagulant mélangé dans la masse solide.

**4.** Procédé selon la revendication 3, dans lequel ledit anticoagulant est une source de citrate non toxique soluble dans l'eau, selon une concentration suffisante.

**5.** Procédé selon la revendication 3, dans lequel ledit agent utile inclut également une source de phosphate non toxique soluble dans l'eau, selon une concentration destinée à améliorer le stockage du sang.

**6.** Procédé selon la revendication 1, 2, 3, 4 ou 5, dans lequel ledit sucre est choisi parmi le glucose, le lactose, le sorbitol, le fructose et le xylitol.

**7.** Procédé selon la revendication 1, 2, 3 ou 5, dans lequel ledit agent utile ajouté est un médicament.

**8.** Procédé selon la revendication 7, dans lequel le médicament est sensible à au moins l'humidité, l'oxygène ou le rayonnement gamma, dans lequel ledit sucre exerce une action de protection sur ledit agent utile avant son contact avec ladite solution médicale.

**9.** Procédé selon la revendication 1, dans lequel ladite solution médicale est une solution parentérale artificielle.

**10.** Procédé selon la revendication 1, dans lequel ledit agent utile est du sucre sensiblement pur qui définit ladite masse.

11. Tablette destinée à une utilisation dans le procédé de la revendication 1, comprenant du glucose sous une forme vitreuse sensiblement anhydre et incluant également une source de citrate non toxique soluble dans l'eau, selon une concentration suffisante pour obtenir un effet d'anticoagulation sur un sang qui passe.

12. Tablette selon la revendication 11, qui inclut une source de phosphate non toxique soluble dans l'eau, selon une concentration destinée à améliorer le stockage du sang.

13. Tablette selon la revendication 12, qui contient approximativement 44 % en poids de dextrose, 46 % en poids de citrate, 6 % en poids d'acide citrique et 4 % en poids de diphosphate de sodium.

14. Tablette selon la revendication 11, dans laquelle ladite tablette contient, mélangées avec elle, de 6 à 10 % en poids de citrate de sodium et de 4 à 7 parties en poids d'acide acétylsalicylique pour 25 parties en poids de dextrose présent.

15. Tablette destinée à une utilisation dans le procédé de la revendication 1, comprenant du sorbitol selon une forme vitreuse sensiblement anhydre, ladite tablette contenant, mélangé à elle, un peptide sous forme de poudre.

FIG. 1

FIG. 2